(19) Europäisches Patentamt
European Patent Office
Office européen des brevets

(11) Numéro de publication: **0 283 364**
**B1**

(12) # FASCICULE DE BREVET EUROPEEN

(45) Date de publication du fascicule du brevet: **09.01.91**

(51) Int. Cl.$^5$: **C 07 C 69/753,**
C 07 C 67/343, C 07 C 69/38

(21) Numéro de dépôt: **88400457.3**

(22) Date de dépôt: **29.02.88**

(54) **Procédé de préparation de monoesters ou diesters de l'acide endoéthano-9,10 dihydro-9,10 anthracène dicarboxylique-11,11, nouveaux monoesters ou diesters ainsi préparés et utilisation de ceux-ci pour la préparation de méthylidène-malonates symétriques ou asymétriques.**

(30) Priorité: **05.03.87 FR 8702991**

(43) Date de publication de la demande:
**21.09.88 Bulletin 88/38**

(45) Mention de la délivrance du brevet:
**09.01.91 Bulletin 91/02**

(84) Etats contractants désignés:
**AT BE CH DE ES FR GB GR IT LI LU NL SE**

(56) Documents cités:
**GB-A-1 073 917**
**US-A-4 013 703**
**US-A-4 139 711**

(73) Titulaire: **LABORATOIRES UPSA**
**1 bis, rue du Docteur Camille Bru**
**F-47000 Agen (FR)**

(72) Inventeur: **Bru-Magniez, Nicole**
**24, avenue Raphael**
**F-75016 Paris (FR)**
Inventeur: **De Cock, Christian**
**56, rue Berckmans**
**Saint Gilles (BE)**
Inventeur: **Poupaert, Jacques**
**22, rue du Palier**
**Ottignies-Louvain la Neuve (BE)**
Inventeur: **De Keyser, Jean-Luc**
**Sneppenlaan 16**
**B-1980 Tervuren (BE)**
Inventeur: **Dumont, Pierre**
**Avenue Moine Olbert 13**
**B-5800 Gembloux (BE)**

(74) Mandataire: **Portal, Gérard et al**
**Cabinet Beau de Loménie 55, rue d'Amsterdam**
**F-75008 Paris (FR)**

**EP 0 283 364 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne un procédé de préparation de monoesters ou diesters de l'acide endoéthano-9,10 dihydro-9,10 anthracène dicarboxylique-11,11, nouveaux monoesters ou diesters ainsi préparés et utilisation de ceux-ci pour la préparation de méthylidènemalonates symétriques ou asymétriques.

Plus particulièrement, les dérivés mono ou diesters selon l'invention permettent de préparer des méthylidènemalonates de formule (I) suivante:

$$CH_2 = C(COOR^1)\ (COOR^2) \qquad (I)$$

où $R^1$ et $R^2$ représentent des groupements alkyles linéaires ou ramifiés de 1 à 6 atomes de carbone, alicycliques ayant de 3 à 6 atomes de carbone, alcényles ayant de 2 à 6 atomes de carbone définis dans leur variété cis ou trans, ou encore alcynyles ayant de 2 à 6 atomes de carbone, fonctionnalisés éventuellement par des groupements tels que éther, époxyde, halogéno, cyano, ester, aldéhyde ou cétone, aryle . . .

L'intérêt des composés de formule (I) précitée est bien connu tant en synthèse organique qu'en chimie des polymères.

On a déjà décrit de nombreux procédés permettant de préparer des méthylidènemalonates de formule similaire à la formule (I) ci-dessus.

Par exemple, un procédé de base consiste à faire réagir le malonate de diéthyle avec le formaldéhyde dans l'acide acétique glacial et en présence de catalyseurs à base d'acétate métallique pour produire le méthylidènemalonate de diéthyle par une distillation après avoir filtré le catalyseur et séparé le solvant.

On peut ensuite utiliser le méthylidènemalonate pour une polymérisation (Chemical Abstracts 1953, vol. 49, abrégé 6836d). La même réaction de base est décrite dans Chemical Abstracts, vol. 76, 1972, abrégé 139905m. Pour la polymérisation voir "Die Makromolekulare Chemie 107 (1967), p. 4—5.

Cependant, dans les conditions habituelles de cette décomposition thermique du composé hydroxylé initialement formé par la réaction entre le malonate et le formaldéhyde, le méthylidénemalonate (I) précité obtenu polymérise, excepté dans le cas où $R^1$ et $R^2$ sont représentés par le groupe t-butyle (voir P. BALLESTEROS, B. W. ROBERTS et J. WANG, J. Org. Chem. *48*, 3603—3605 (1983)).

Par ailleurs, on a déjà proposé la préparation de méthylidènemalonates symétriques ou asymétriques en faisant réagir un diester malonique avec le formaldéhyde en présence d'un diène pour aboutir à un produit d'addition Diels-Alder qui est ensuite soumis à pyrolyse pour donner le méthylidènemalonate.

Ainsi, dans le document GB—A—1 560 323 EASTMAN KODAK, on utilise comme diène, un diène linéaire comme un pentadiène substitué, un hexadiène, l'isoprène, le 1,3-butadiène non substitué ou substitué, et le produit d'addition intermédiaire est ensuite pyrolysé à 600°C pour libérer le méthylidénemalonate.

D'autre part, dans le document DE—C—27 34 082 PONTICELLO, on décrit aussi la préparation de méthylidènemalonates asymétriques en procédant à une réaction de type Diels-Alder entre un acrylate de méthyle et le cyclopentadiène de manière à faire un produit d'addition intermédiaire qui est ensuite soumis à diverses réactions chimiques pour obtenir un diester avant décomposition par pyrolyse pour fournir le méthylidènemalonate diester asymétrique.

D'autre part, on connaît encore la synthèse d'alpha-cyanoacrylate d'alcoyle par réaction d'un ester d'acide cyanoacrylique avec un diène conjugué, exemplifié par l'anthracène, pour former le produit d'addition Diels-Alder qui est ensuite hydrolysé (voir le document US—A—3 975 422 ou GIRAL, Annal Pharmaceutiques Françaises 1985, 43, No 5, pages 439—449 ou encore PONTICELLO US—4 056 543); mais ici, l'ester d'acide cyanoacrylique contient au départ une insaturation qui est utilisée pour l'addition avec l'anthracène. Une telle réaction d'addition est très ancienne et était déjà décrite par BACHMAN et TANNER dans J. Org. Chem. *4*, (1939) p. 500. Elle a été utilisée pour la purification du méthylidènemalonate, préalablement formé par addition avec le cyclopentadiène ou le norbornène (voir C.A., vol 95, 1981, abrégé 168570w).

Ainsi, pour l'homme du métier, l'utilisation de l'anthracène n'a été décrite que pour former un produit d'addition avec un composé insaturé, à savoir le méthylidènemalonate de diéthyle préalablement formé ou un ester d'acide cyanoacrylique.

D'autre part, l'état de la technique enseigne à l'homme du métier, par le document GB—A—1 560 323 précité, le piègeage du méthylidènemalonate in situ lors de la réaction d'un malonate avec le formaldéhyde à l'aide d'un linéaire. Ainsi, pour l'homme du métier, un tel piègeage in situ à l'aide d'un diène cyclique ne lui paraissait pas possible sans doute en raison des conditions réactionnelles défavorables.

Or, il vient d'être découvert, de manière totalement inattendue, allant à l'encontre de l'enseignement de l'état de la technique, que l'anthracène constituait un diène conjugué permettant de piéger très efficacement (c'est-à-dire avec d'excellents rendements), de manière très simple in situ, le méthylidènemalonate formé in situ par la réaction entre le malonate et le formaldéhyde, le produit d'addition formé étant facilement cristallisable.

En résumé, toutes les voies de synthèse actuelles présentent des inconvénients majeurs qui rendent mal aisé, voire impossible, leur transposition au plan industriel.

Ainsi, la formation directe de méthylidènemalonate ne peut pas être utilisée puisqu'elle conduit à une

polymérisation inévitable du méthylidènemalonate formé.

Dans les cas de préparation d'un produit d'addition intermédiaire, celui-ci est souvent difficilement filtrable et purifiable par recristallisation et toujours contaminé par des quantités non négligeables de diènes conjugués utilisés pour la réaction de Diels-Alder, cette contamination affectant l'étape ultérieure de thermolyse ou d'hydrolyse. Une purification par distillation sous vide poussée est donc dans ce cas nécessaire dans les procédés antérieurs.

En outre, les rendements sont en général relativement modestes et le nombre d'étapes de formation du méthylidènemalonate est relativement élevé surtout dans le cas de la formation des esters asymétriques.

La présente invention a donc pour but de résoudre le nouveau problème technique consistant en la fourniture d'un nouveau procédé de synthèse de methylidènemalonate utilisable sur le plan industriel, d'une grande simplicité, fiable, utilisant des réactifs peu coûteux, avec un minimum d'étapes, de préférence seulement deux étapes principales avec l'obtention de produits d'une grande pureté en des rendements élevés, et permettant la préparation d'une large gamme de produits, y compris ceux comportant des groupes réactifs sur les substituants esters $R^1$ et $R^2$ précités.

La présente invention a encore pour but principal de résoudre le nouveau problème technique consistant en la formation de methylidènemalonates asymétriques, et en particulier ceux dont un des esters a été fonctionnalisé par au moins un groupement éther, époxyde, halogène, cyano, ester, aldéhyde, acétone, aryle, etc.

La présente invention a encore pour but de résoudre le nouveau problème technique consistant en la fourniture d'un nouveau procédé de synthèse de methylidènemalonate par la formation d'un produit d'addition ou adduit, type Diels-Alder d'une manière très simple, très rapide, en un rendement élevé, ce composé d'addition étant avantageusement capable de subir une hémihydrolyse, ce qui n'était pas possible précédemment, permettant la préparation à volonté de produits d'addition asymétriques par alkylation au moyen d'un halogénure approprié.

La présente invention a encore pour but de résoudre le nouveau problème technique consistant en la formation de nouveaux produits d'addition intermédiaires utilisables pour la synthèse de methylidènemalonate, capables d'être aisément isolables par cristallisation, en une haute pureté, les faibles quantités de produits contaminants n'affectant en rien leur capacité de formation ultérieure de methylidènemalonates, ces produits d'addition étant des esters symétriques ou avantageusement asymétriques, ou des monoesters.

De préférence, ces produits d'addition permettent de conduire au methylidènemalonate par thymolyse à des températures notablement plus faibles que dans le cas d'autres produits d'addition connus.

En outre, l'invention fournit aussi une solution qui utilise des solvants à faibles toxicités, facilitant l'extraction et l'isolement des produits d'addition.

Tous ces nouveaux problèmes techniques sont résolus pour la première fois par la présente invention, d'une manière trés simple, rapide, avec la formation de produits d'addition et de methylidènemalonate en une haute pureté, et avec des rendements élevés.

Ainsi la présente invention, selon un premier aspect, fournit un procédé de préparation de mono ou de diesters de l'acide endoéthano-9,10 dihydro-9,10 anthracène dicarboxylique-11,11 de formule (II) suivante:

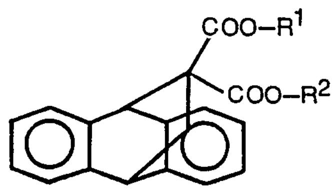

(II)

dans laquelle $R^1$ et $R^2$ peuvent être identiques ou différents et représenter H ou un atome de métal alcalin ou alcalino-terreux, notamment sodium, potassium, un groupement alkyle linéaire ou ramifié ayant de 1 à 6 atomes de carbone, alicyclique ayant de 3 à 6 atomes de carbone, alcényle ayant de 2 à 6 atomes de carbone définis dans leur variété cis ou trans, ou encore alcynyle ayant de 2 à 6 atomes de carbone, éventuellement fonctionnalisé par un ou plusieurs groupements tels que éther, époxyde, halogéno, cyano, ester, aldéhyde, cétone, aryle . . . , $R^1$ et $R^2$ ne peuvent pas être simultanément H, caractérisé en ce qu'on fait réagir un ester malonique correspondant avec le formaldéhyde en présence d'anthracène, de manière à former ledit mono ou diester, sous forme de produits d'addition, et de préférence ce mono ou diester est séparé du milieu réactionnel, avantageusement pour être obtenu sous forme de produit cristallisé.

Selon un mode de réalisation avantageux de ce procédé, la réaction a lieu en milieu solvant non aqueux, en présence d'un catalyseur, de préférence choisi parmi l'acétate de cuivre (III), l'acétate de potassium ou leurs mélanges.

Avantageusement, ce solvant non aqueux est choisi parmi un solvant non miscible à l'eau ou avantageusement un solvant miscible à l'eau. Parmi ces solvants, on peut citer: l'acide acétique, l'anhydride acétique, le benzène, le bromobenzène, le xylène, le toluène, le diméthylformamide (DMF), le diméthylsulfoxyde (DMSO), une cétone comme la diméthylcétone, l'éthylméthylcétone; l'acétonitrile, le dioxane, la N-méthylpyrrolidone (NMP); ou un mélange quelconque d'au moins 2 ou 3 de ces solvants.

Selon un mode de réalisation avantageux, la synthèse des produits d'addition monoesters est obtenue à partir des dérivés diesters, de préférence par réaction dans un solvant alcoolique, avec un sel de métal alcalin ou alcalino-terreux, et notamment un hydroxyde de sodium ou de potassium.

Selon un mode de réalisation également avantageux, on prépare les produits d'addition diesters asymétriques à partir du produit d'addition monoester par réaction avec un produit halogéné dont le radical est destiné à constituer un deuxième radical ester différent du premier radical ester.

Selon une variante de réalisation avantageuse du procédé, la réaction est réalisée en système clos, en particulier en autoclave ou tube de Carius.

Egalement, la présente invention fournit, selon un deuxième aspect, de nouveaux monoesters et diesters de l'acide endoéthano-9,10 dihydro-9,10 anthracène dicarboxylique-11,11 répondant avantageusement à la formule (II) chimique développée suivante:

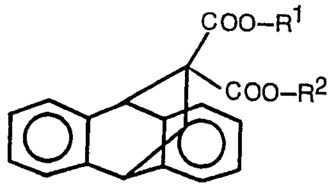

(II)

dans laquelle $R^1$ et $R^2$ peuvent être identiques ou différents et représenter H ou un atome de métal alcalin ou alcalino-terreux, notamment sodium, potassium, un groupement alkyle linéaire ou ramifié ayant de 1 à 6 atomes de carbone, alicyclique ayant de 3 à 6 atomes de carbone, alcényle ayant de 2 à 6 atomes de carbone définis dans leur variété cis ou trans, ou encore alcynyle ayant de 2 à 6 atomes de carbone, éventuellement fonctionnalisé par un ou plusieurs groupements tels que éther, époxyde, halogèno, cyano, ester, aldéhyde, cétone, aryle . . . , $R^1$ et $R^2$ ne peuvent pas être simultanément H ou un groupe éthyle.

En particulier, l'invention couvre les mono et diesters suivants, constituant des produits d'addition intermédiaires pour la préparation de methylidènemalonates:

— di-n-propoxycarbonyl-11,11 endoéthano-9,10 dihydro-9,10 anthracène
— di-n-butoxycarbonyl-11,11 endoéthano-9,10 dihydro-9,10 anthracène-n-butoxycarbonyl, n-pentoxycarbonyl-11,11 endoéthano-9,10 dihydro-9,10 anthracène
— éthoxycarbonyl, éthoxycarbonylméthoxycarbonyl-11,11 endoéthano-9,10 dihydro-9,10 anthracène
— diméthoxycarbonyl-11,11 endoéthano-9,10 dihydro-9,10 anthracène
— méthoxycarbonyl, n-butoxycarbonyl-11,11 endoéthano-9,10 dihydro-9,10 anthracène
— méthoxycarbonyl, n-hexyloxycarbonyl-11,11 endoéthano-9,10 dihydro-9,10 anthracène
— méthoxycarbonyl, benzyloxycarbonyl-11,11 endoéthano-9,10 dihydro-9,10 anthracène
— diéthoxycarbonyl-11,11 endoéthano-9,10 dihydro-9,10 anthracène
— éthoxycarbonyl, n-propoxycarbonyl-11,11 endoéthano-9,10 dihydro-9,10 anthracène
— éthoxycarbonyl, n-butoxycarbonyl-11,11 endoéthano-9,10 dihydro-9,10 anthracène
— éthoxycarbonyl, allyloxycarbonyl-11,11 endoéthano-9,10 dihydro-9,10 anthracène
— éthoxycarbonyl, n-propynyl-3-oxycarbonyl-11,11 endoéthano-9,10 dihydro-9,10 anthracène
— éthoxycarbonyl, méthoxyméthoxycarbonyl-11,11 endoéthano-9,10 dihydro-9,10 anthracène
— éthoxycarbonyl, éthoxyéthoxycarbonyl-11,11 endoéthano-9,10 dihydro-9,10 anthracène
— éthoxycarbonyl, éthoxycarbonylpropoxycarbonyl-11,11 endoéthano-9,10 dihydro-9,10 anthracène
— éthoxycarbonyl 2',3'-époxypropoxycarbonyl-11,11 endoéthano-9,10 dihydro-9,10 anthracène
— éthoxycarbonyl, propan-ol-3, yloxycarbonyl-11,11 endoéthano-9,10 dihydro-9,10 anthracène
— n propoxycarbonyl, n-butoxycarbonyl-11,11 endoéthano-9,10 dihydro-9,10 anthracène
— di-iso-propoxycarbonyl-11,11 endoéthano-9,10 dihydro-9,10 anthracène
— di-iso-butoxycarbonyl-11,11 endoéthano-9,10 dihydro-9,10 anthracène
— di-n-pentoxycarbonyl-11,11 endoéthano-9,10 dihydro-9,10 anthracène
— di-allyloxycarbonyl-11,11 endoéthano-9,10 dihydro-9,10 anthracène
— triméthylène-1',3'-dioxycarbonyl-11,11 endoéthano-9,10 dihydro-9,10 anthracène.

Enfin, l'invention, selon un troisième aspect, couvre encore l'utilisation des monoesters ou diesters pour la préparation de methylidènemalonates, par tout traitement connu en soi, comme un traitement thermique, thermolyse ou pyrolyse, ou encore hydrolyse.

D'autres buts, caractéristiques et avantages de l'invention apparaîtront clairement à la lumière de la description explicative qui va suivre, donnant quelques exemples de préparation donnés simplement à titre d'illustration et qui ne sauraient donc en aucune façon limiter la portée de l'invention.

### Exemple 1

Préparation de di-iso-propyloxycarbonyl-11,11 endoéthano-9,10 dihydro-9,10 anthracène (II, $R^1 = R^2 = iso\text{-}C_3H_7$)

188 g (1 mole) de malonate de di-iso-propyle sont chauffés sous agitation au moyen d'un bain d'huile à 90—100°C en présence de 60 g (2 moles) de paraformaldéhyde, de 178 g (1 mole) d'anthracène, de 10 g d'acétate de cuivre II et de 10 g d'acétate de potassium dans 500 ml d'acide acétique et 500 ml de bromobenzène. La température est maintenue durant 2 heures à 90—100°C. La température du bain d'huile est ensuite augmentée progressivement afin de distiller initialement un mélange azéotrope composé d'eau, de

bromobenzène et d'acide acétique et ensuite l'acide acétique restant. La distillation est arrêtée lorsque les acétates de cuivre II et de potassium précipitent. Le milieu de réaction préalablement refroidi à 60°C est déversé dans 1 l de toluène. Ce mélange refroidi à 10°C est essoré sur buchner et le filtrat évaporé à sec. Le résidu solide est recristallisé dans l'éthanol. Le produit ainsi obtenu présente une pureté de 94%; il est contaminé (6%) par de l'anthracène. Le produit purifié par chromatographie sur colonne de gel de silice (hexane: isopropanol;, 95:5, v/v) présente un point de fusion de 136—7°C; rendement: 72% (278,16 g). Ce composé s'analyse correctement pour la formule $C_{24}H_{26}O_4$.

Exemple 2

Préparation du di-allyloxycarbonyl-11,11-endoéthano-9,10 dihydro-9,10 anthracène (II, $R^1 = R^2\ CH_2—CH=CH_2$).

On procéde comme dans l'exemple 1, mais en mettant en réaction les quantités suivantes de réactifs: 46 g (0,25 mole) de malonate de diallyle, 15 g (0,5 mole) de paraformaldéhyde, 44,5 g (0,25 mole) d'anthracène, 5 g d'acétate de cuivre II et 5 g d'acétate de potassium dans 120 ml de bromobenzène et 120 ml d'acide acétique. Après recristallisation, le produit présente un point de fusion de 85—86°C et le rendement est de 45% (41,35 g). Ce composé s'analyse correctement pour la formule $C_{24}H_{22}O_4$.

Les dérivés suivants ont également été préparés selon ce procédé:

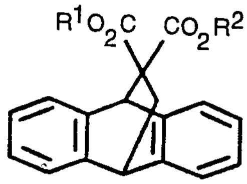

| $R^1$ | $R^2$ | Rendement % | Fusion °C | Analyse élémentaire |
|---|---|---|---|---|
| $CH_3$ | $CH_3$ | 53 | 161-162 | $C_{20}H_{18}O_4$ |
| $CH_3$ | $nC_4H_9$ | 51 | 80-82 | - |
| $CH_3$ | $nC_6H_{13}$ | 53 | 74-75 | - |
| $CH_3$ | $CH_2C_6H_5$ | 42 | 109-112 | - |
| $C_2H_5$ | $C_2H_5$ | 75 | 130-131 | $C_{22}H_{22}O_4$ |
| $C_2H_5$ | $nC_3H_7$ | 82 | 107-108 | $C_{23}H_{24}O_4$ |
| $C_2H_5$ | $nC_4H_9$ | 46 | 91-92 | - |
| $C_2H_5$ | $CH_2-CH=CH_2$ | 84 | 88-89 | $C_{22}H_{22}O_4$ |
| $C_2H_5$ | $CH_2-C\equiv CH$ | 62 | 60-61 | - |
| $C_2H_5$ | $CH_2OCH_3$ | 75 | 106-107 | $C_{22}H_{22}O_5$ |
| $C_2H_5$ | $C_2H_4OC_2H_5$ | 47 | 42-46 | - |
| $C_2H_5$ | $CH_2CO_2C_2H_5$ | 42 | 76-77 | - |
| $C_2H_5$ | $(CH_2)_3CO_2C_2H_5$ | 67 | 83-84 | - |
| $C_2H_5$ | O<br>$CH_2CH-CH_2$ | 66 | 114-115 | $C_{23}H_{22}O_5$ |

| $R^1$ | $R^2$ | Rendement % | Fusion °C | Analyse élémentaire |
|---|---|---|---|---|
| $C_2H_5$ | $CH_2CH_2CH_2$-OH | 53 | 95-98 | - |
| $nC_3H_7$ | $nC_3H_7$ | 72 | 104-106 | $C_{24}H_{26}O_4$ |
| $nC_3H_7$ | $nC_4H_9$ | 47 | 91-92 | - |
| iso $C_3H_7$ | iso$C_3H_7$ | 72 | 136-137 | $C_{24}H_{26}O_4$ |
| $nC_4H_9$ | $nC_4H_9$ | 55 | 91-92 | $C_{26}H_{30}O_4$ |
| iso$C_4H_9$ | iso$C_4H_9$ | 52 | 94-95 | $C_{26}H_{30}O_4$ |
| $nC_4H_9$ | $nC_5H_{11}$ | 53 | 77-79 | - |
| $nC_5H_{11}$ | $nC_5H_{11}$ | 45 | 75-76 | - |
| $CH_2$ $CH=CH_2$ | $CH_2CH=CH_2$ | 41 | 85-86 | - |
| $-CH_2-CH_2-CH_2-$ | | 53 | 115-118 | - |

Exemple 3

Synthèse du sel potassique de carboxylate-11 éthoxycarbonyl-11 endoéthano-9,10 dihydro-9,10 anthracène (III, $R^1 = C_2H_5$, $R^2 = K$).

A une solution portée à 65°C de 100 g (0,286 mole) de diéthoxycarbonyl-11,11 endoéthano-9,10 dihydro-9,10 anthracène dans 400 ml d'éthanol absolu, on ajoute goutte à goutte et sous agitation une solution de 18,6 g (0,324 mole) d'hydroxyde de potassium dans 400 ml d'éthanol absolu. Aprés 4 heures, le mélange réactionnel est refroidi à température ordinaire et le sel potassique qui a précité est essoré et lavé par de l'éhter diéthylique. Après séchage sous vide à température ordinaire, on obtient 92 g (rendement: 90%) d'une poudre blanche.

Exemple 4

Synthèse de l'allyloxycarbonyl-11 endoéthano-9,10 dihydro-9,10 anthracène (II, $R^1 = CH_2—CH = CH_2$, $R^2 = C_2H_5$).

On met en réaction 20 g (0,0555 mole) de (III) et 8,4 g (0,0555 mole) de bromure d'allyle dans 250 ml de diméthylformamide anhydre. Le milieu de réaction est chauffé à 80°C, agité durant 2 heures, dilué dans 2 l d'eau et essoré; le précipité est lavé à l'eau et recristallisé dans l'éthanol pour donner 17 g (85%) de point de fusion 88—89°C. Ce composé s'analyse correctement pour la formule $C_{23}H_{22}O_4$.

## Exemple 5

Synthèse de l'époxy-2',3' propyloxycarbonyl-11 éthoxycarbonyl-11 endoéthano-9,10 dihydro-9,10 anthracène (II,

$$R^1 = CH_2\text{-}\overbrace{CH\text{-}CH_2\text{-}O}, \quad R^2 = C_2H_5).$$

On procéde de la même façon que dans l'exemple 4 en mettant en réaction les quantités suivantes: 20 g (0,0555 mole) de (III), 8,9 g (0,065 mole) d'épibromhydrine. Le produit est obtenu avec un rendement de 70% après recristallisation dans l'éthanol (Fusion: 114—115°C). Ce compose s'analyse correctement pour la formule $C_{23}H_{22}O_5$.

## Exemple 6

Synthèse de: triméthylène-1,3 di (oxycarbonyl)-11,11 endoéthano-9,10 dihydro-9,10 anthracène (II, $R^1$ $R^2$ = $CH_2$—$CH_2$—$CH_2$—) on met en réaction 5 g (0,013 mole) d'éthyloxypropanol-3 yloxy) carbonyl-11,11 endoéthano-9,10 anthracène (II: $R^1$ = $C_2H_5$, $R^2$ = —$C_2H_4$—$CH_2OH$); obtenu par alkylation du sel potassique (III: $R^1$ = $C_2H_5$, $R^2$ = K) par du bromo-3 propanol-1 dans du DMF, dans 50 ml de xylène (sec) avec une quantité catalytique de NaH (60% en dispersion dans l'huile de paraffine). Le ballon est surmonté d'un vigreux, le mélange est chauffé afin de distiller l'azéotrope xylène-éthanol. Après évaporation du solvant, le résidu solide est recristallisé dans l'éthanol. Rendement: 53% (2,52 g), point de fusion: 115—118°C.

Les exemples 7 à 10 ci-après concernent des variantes de réalisation avantageuses du procédé de base de préparation du produit d'addition (II).

## Exemple 7

*Utilisation de xylène en lieu et place du bromobenzène*

On obtient une réduction du coût et une moindre toxicité.

Quantité de solvant: 1,5 volume de xylène
1 volume d'acide acétique

Les autres conditions sont identiques à celles décrites pour le bromobenzène à l'exemple 1, mais on utilise, comme produit de départ, le malonate de di-n-butyle en lieu et place du malonate de di-iso-propyle.

On obtient le di-n-butyloxycarbonyl-11,11 endoéthano-9,10 dihydro-9,10 anthracène (II: $R_1$ = $R^2$ = $nC_4H_9$); Rendement 55%, point de fusion: 91—92°C.

## Exemple 8

*Utilisation d'un solvant miscible à l'eau*

La méthode d'isolement est plus aisée.

Une gamme de solvants, assurant des conditions thermiques semblables au procédé xylène (ou bromobenzène) mais miscibles à l'eau a été étudiée. La distillation de solvants à haut point d'ébullition est ainsi évitée, le produit de réaction étant isolé par filtration après addition d'eau.

Le mélange réactionnel est refroidi après un temps de chauffage de 3 heures à 140°C. Ce mélange est déversé dans l'eau, le solide est filtré. La suite du procédé est identique à celle de l'exemple 1.

Les rendements donnés dans le tableau sont ceux obtenus pour la synthèse de diéthyloxycarbonyl-11,11 endoéthano-9,10 dihydro-9,10 anthracène (II: $R^1 = R^2 = C_2H_5$) à partir du malonate de diéthyle.

| Solvants (proportions v/v) | Rendement % |
|---|---|
| DMF/Acide Ac./$C_6H_6$<br>9 9 2 | 24 |
| DMSO/Acide Ac.<br>1 1 | 8 |
| DMSO/Acide Ac./Dioxane<br>2 0,8 2 | 25 |
| DMSO/Acide Ac./Toluène<br>2 1 2 | 28 |
| NMP/Acide Ac./Xylène<br>2 2 0,8 | 51 |

Exemple 9

*Utilisation d'un autoclave ou d'un tube de Carius.*

Procédé en système clos

Exemple: synthèse du diéthyloxycarbonyl-11,11 endoéthano-9,10 dihydro-9,10 anthracène.

Un autoclave (contenance: 100 ml) est chargé avec 17 g d'anthracène, 6 g de paraformaldéhyde et de 16 g de malonate de diéthyle.

Le catalyseur est un mélange de 0,5 g d'acétate cuivreux et 0,5 g d'acétate de potassium.

Le solvant (50 ml) est un mélange d'acide acétique et de benzène dans un rapport 2,5/7,5 (v/v).

L'autoclave est fermé, puis plongé dans un bain d'huile chauffé à 90—100°C durant 2 heures. La température du bain est portée progressivement à 140—150°C, ceci durant 3 heures. L'autoclave est refroidi à température ambiante puis ouvert. Le mélange réactionnel est repris par 100 ml de benzène. A la solution, nous ajoutons du $CaCl_2$ (anhydre). Après filtration, les solvants, sont évaporés, le résidu solide est recristallisé dans l'éthanol. Rendement: 67% point de fusion: 127—129°C.

Le tableau suivant reprend les différentes conditions examinées. Les quantités de réactifs sont identiques à celles décrites dans l'exemple ci-dessus (sauf indication contraire).

| Solvants/(proportions v/v) | Catalyseur | Rendement |
|---|---|---|
| Xylène/Acide acétique<br>1 1 | $(Ac)_2Cu$<br>+ AcK | 62 % |
| Benzène/Acide acétique<br>1 1 | " | 41 % |
| Benzène/Acide acétique<br>7/8 1/8 | " | 49 % |
| Benzène/Acide acétique<br>15/16 1/16 | " | 46 % |
| Benzène/Acide acétique<br>3/4 1/4 | " | 67 % |
| Benzène/Acide acétique<br>3/4 1/4<br>(Chauffage durant 12 h) | " | 46 % |
| Xylène/Acide acét./Anhydride acét.<br>2 2 1 | " | 32 % |
| Xylène/Anhydride acétique<br>2 1 | " | 32 % |
| Diméthylcétone/Acide acétique<br>3/4 1/4 | " | 56 % |
| Ethylméthylcétone/Acide acétique<br>3/4 1/4 | " | 63 % |
| Acétonitrile/Acide acétique<br>3/4 1/4 | " | 58 % |
| Dioxane/Acide acétique<br>3/4 1/4 | " | 56 % |
| Dioxane/Acide acétique<br>3/4 1/4 | $(ClCH_2CO_2)_2Mg$<br>1g | 31 % |
| Diméthylcétone/Acide acétique<br>3/4 1/4 | " | 26 % |
| Xylène/Acide acétique/Anhyd. acét.<br>5 5 2 | " | 56 % |

| Solvants/(proportions v/v) | Catalyseur | Rendement |
|---|---|---|
| Benzène/Acide acétique 3/4 1/4 | $(ClCH_2CO_2)_2Mg$ 1g + $(Ac)_2Cu$ 0,5 g AcK 0,5 g | 60 % |
| Méthyléthylcétone/Acide acétique 3/4 1/4 | $(Ac)_2Cu$ 2,5 g | 75 % |

Exemple 10

Synthèse du di-iso-propyloxycarbonyl-1,1 éthène (I, $R^1 = R^2 = iso\text{-}C_3H_7$).

Dans 250 ml d'huile minérale, on disperse sous bonne agitation et sous courant d'azote sec 50 g de l'adduit (0,132 mole) décrit dans l'exemple 1 et 10,37 g (0,105 mole) d'anhydride maléique. On amène progressivement cette suspension à 200—220°C. Cette température est maintenue durant 45 minutes; après quoi, le mélange de réaction est refroidi à température ordinaire, mis sous vide (0,1 Torr) et distillé. On recueille la fraction de point d'ébullition 40°C. Rendement: 64% (16,89 g). Pureté 99% (contaminant: 1% d'anhydride maléique). Spectre de masse (70 eV), ionisation chimique (isobutane): 201 (M+1), 159, 117.

Exemple 11

Synthèse de l'allyloxycarbonyl-1 éthyloxycarbonyl-1 éthène (I, $R^1 = C_2H_5$, $R^2 = CH_2—CH{=}CH_2$).

Suivant le mode opératoire décrit à l'exemple 10, on met en réaction dans 70 ml d'huile minérale, 10 g de l'adduit (0,0276 mole) dans l'exemple 4 et 2,16 g (0,022 mole) d'anhydride maléique. Le produit obtenu après distillation sous 0,25 Torr à un point d'ébullition de 53°C, le rendement est de 48% (2,4 g) et la pureté de 99% (contaminant: 1% d'anhydride maléique).

Les exemples 10 et 11 sont représentatifs de la méthode générale de thermolyse des adduits (II) en présence d'anhydride maléique.

Selon cette méthode, on peut par exemple préparer:

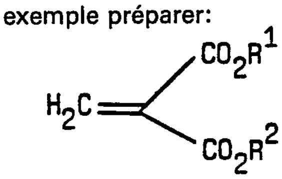

| $R^1$ | $R^2$ | Rendement | Ebullition °C (Torr) |
|---|---|---|---|
| $CH_3$ | $CH_3$ | 54 | 80-82 (6) |
| $CH_3$ | $nC_4H_9$ | 75 | 65-68 (0,4) |
| $CH_3$ | $nC_6H_{13}$ | 77 | 80-85 (0,1) |
| $C_2H_5$ | $C_2H_5$ | 67 | 60-61 (0,25) |
| $C_2H_5$ | $C_3H_7$ | 63 | 52-55 (0,3) |
| $C_2H_5$ | $C_4H_9$ | 71 | 62-63 (0,2) |
| $C_2H_5$ | $CH_2\text{-}CH{=}CH_2$ | 48 | 53-55 (0,25) |

| $R^1$ | $R^2$ | Rendement | Ebullition °C (Torr) |
|---|---|---|---|
| $C_2H_5$ | $CH_2$-C= CH | 20 | 65-77 (0,3) |
| $C_2H_5$ | $C_2H_4OC_2H_5$ | 43 | 82-84 (0,2) |
| $C_2H_5$ | $CH_2CO_2C_2H_5$ | 62 | 98-99 (0,1) |
| $C_2H_5$ | $CH_2CH_2CH_2CO_2C_2H_5$ | 32 | 86-89 (0,06 ) |
| $nC_3H_7$ | $nC_3H_7$ | 81 | 77-78 (0,2) |
| $nC_3H_7$ | $nC_4H_9$ | 54 | 78-80 (0,1) |
| iso$C_3H_7$ | iso$C_3H_7$ | 64 | 40-42 (0,1) |
| $nC_4H_9$ | $nC_4H_9$ | 68 | 76-80 (0,01) |
| $nC_4H_9$ | $nC_5H_{11}$ | 78 | 95-96 (0,1) |
| $nC_5H_{11}$ | $nC_5H_{11}$ | 46 | 99-101 (0,05) |
| isobutyl | isobutyl | 61 | 64-65 (0,02) |
| allyle | allyle | 48 | 67-68 (0,3) |

On comprend ainsi que la présente invention permet de préparer des produits d'addition avec l'anthracène d'une manière très simple, très rapidement, avec une pureté élevée ainsi que des rendements élevés. En outre, on peut préparer des produits d'addition asymétriques car les produits d'addition de base peuvent subir une hémihydrolyse en milieu basique, pour donner un monosel alcalin ou alcalino-terreux d'alkoxy carbonyl-11 carboxylate-11 endoéthano-9,10 dihydro-9,10 anthracène, qui, par alkylation au moyen d'un halogénure approprié $R^3$—X dans le diméthylformamide fournit le produit d'addition asymétrique, conformément au schéma 2 annexé.

Ainsi, le produit d'addition asymétrique traité à environ 220°C dans une huile minérale en présence d'anhydride maléique ou par tout autre moyen de thermolyse ou de pyrolyse fournit l'oléfine correspondante, c'est-à-dire le methylidènemalonate correspondant.

Ainsi, le procédé selon l'invention permet de préparer des methylidènemalonates à partir de malonates esters en deux étapes essentielles, à savoir dans une première étape la réaction de l'ester malonique avec le formaldéhyde en présence d'anthracène, conformément au schéma 1 ci-après, et dans une deuxième étape, le traitement thermique du produit d'addition obtenu par la première étape pour former le methylidènemalonate correspondant, avantageusement en présence d'anhydride maléique de manière à séparer l'anthracène encore sous la forme d'un produit d'addition (Schéma 1).

La présente invention comprend donc tous les moyens constituant des équivalents techniques des moyens décrits et revendiqués dans les revendications.

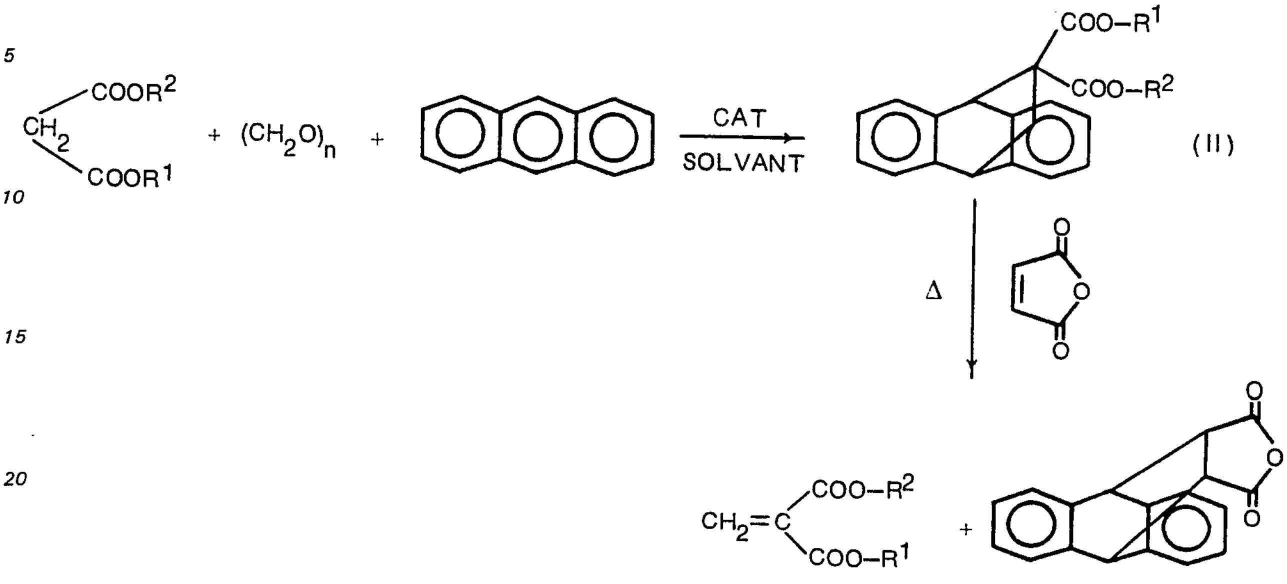


(I)

SCHEMA : I

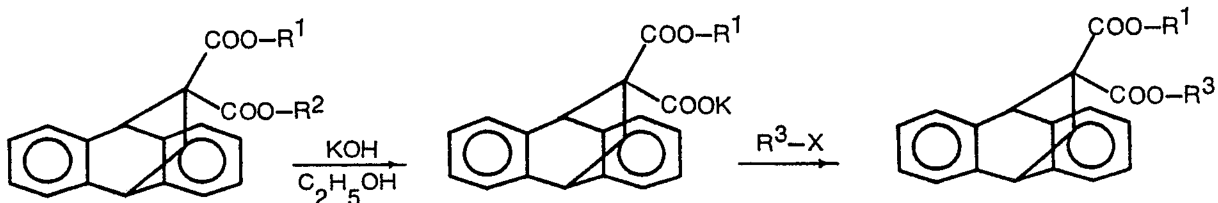


(II) (III) (II)

$R^1 \neq R^3$

$R^1 = R^2$

SCHEMA : 2

**Revendications pour les Etats contractants: DE GB FR IT NL SE CH LI BE AT LU**

1. Procédé de préparation de monoesters ou diesters de l'acide endoéthano-9,10 dihydro-9,10 anthracène dicarboxylique-11,11, de formule (II) suivante:

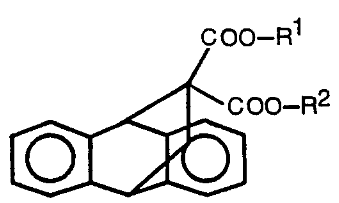

(II)

dans laquelle $R^1$ et $R^2$ peuvent être identiques ou différents et représenter H ou un atome de métal alcalin ou alcalino-terreux, notamment sodium, potassium, un groupement alkyle linéaire ou ramifié ayant de 1 à 6 atomes de carbone, alicyclique ayant de 3 à 6 atomes de carbone, alcényle ayant de 2 à 6 atomes de carbone définis dans leur variété cis ou trans, ou encore alcynyle ayant de 2 à 6 atomes de carbone, éventuellement fonctionnalisé par un ou plusieurs groupements tels que éther, époxyde, halogéno, cyano, ester, aldéhyde, cétone, aryle . . . , où $R^1$ et $R^2$ ne peuvent pas être simultanément H, caractérisé en ce qu'on fait réagir un ester malonique correspondant avec le formaldéhyde en présence d'anthracène, de manière à former ledit mono ou diester et de préférence, on sépare ce mono ou diester du milieu réactionnel, avantageusement pour être obtenu sous forme de produits cristallisés.

2. Procédé selon la revendication 1, caractérisé en ce qu'on réalise la réaction dans un milieu solvant

non aqueux en présence d'un catalyseur de préférence choisi parmi l'acétate de cuivre II et l'acétate de potassium, ou encore de préférence un mélange des deux.

3. Procédé selon la revendication 2, caractérisé en ce que le solvant non aqueux est choisi parmi un solvant non miscible à l'eau ou un solvant miscible àl'eau, notamment l'acide acétique, l'anhydride acétique, le benzène, le bromobenzène, le xylène, le toluène, le diméthylformamide (DMF), le diméthylsulfoxyde (DMSO), une cétone comme la diméthylcétone ou l'éthylméthylcétone, l'acétonitrile, le dioxane, la N-méthylpyrrolidone (NMP); ou un mélange quelconque d'au moins 2 ou 3 de ces solvants.

4. Procédé selon l'une des revendications 1 à 3, caractérisé en ce que l'on prépare le monoester à partir des diesters par une hémihydrolyse en milieu basique pour donner un monosel alcalin ou alcalino-terreux d'alkoxy carbonyl-11, carboxylate-11 endoéthano-9,10 dihydro-9,10 anthracène.

5. Procédé selon la revendication 1 ou 4, caractérisé en ce qu'on prépare les diesters asymétriques en faisant réagir le monoester précité avec un dérivé halogéne approprié, par une réaction d'alkylation.

6. Nouveaux monoesters ou diesters de l'acide endoéthano-9,10 dihydro-9,10 anthracène dicarboxylique-11,11, caractérisé en ce qu'ils répondent à la formule chimique (II) développée suivante:

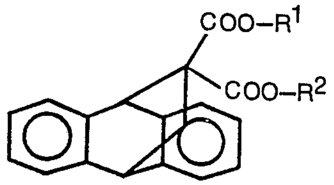

(II)

dans laquelle $R^1$ et $R^2$ peuvent être identiques ou différents et représenter H, ou un atome de métal alcalin ou alcalino-terreux, et notamment du sodium, du potassium, ou un groupement alkyle linéaire ou ramifié ayant de 1 à 6 atomes de carbone, alicycliques ayant de 3 à 6 atomes de carbone, alcényles ayant de 2 à 6 atomes de carbone définis dans leur variété cis ou trans, ou encore alcynyles ayant de 2 à 6 atomes de carbone, éventuellement fonctionnalisés par un ou plusieurs groupements tels que éther, époxyde, halogéno, cyano, ester, aldéhyde, cétone, aryle . . . , où $R^1$ et $R^2$ ne peuvent pas être simultanément H ou éthyle.

7. Nouveaux monoesters ou diesters selon la revendication 6, caractérisés en ce qu'il s'agit des diesters symétriques suivants:
— di-n-propoxycarbonyl-11,11 endoéthano-9,10 dihydro-9,10 anthracène
— di-n-butoxycarbonyl-11,11 endoéthano-9,10 dihydro-9,10 anthracène
— diméthoxycarbonyl-11,11 endoéthano-9,10 dihydro-9,10 anthracène
— diéthoxycarbonyl-11,11 endoéthano-9,10 dihydro-9,10 anthracène
— di-iso-propoxycarbonyl-11,11 endoéthano-9,10 dihydro-9,10 anthracène
— di-iso-butoxycarbonyl-11,11 endoéthano-9,10 dihydro-9,10 anthracène
— di-n-pentoxycarbonyl-11,11 endoéthano-9,10 dihydro-9,10 anthracène
— di-allyloxycarbonyl-11,11 endoéthano-9,10 dihydro-9,10 anthracène
— triméthylène-1',3'-dioxycarbonyl-11,11 endoéthano-9,10 dihydro-9,10 anthracène.

8. Nouveaux monoesters ou diesters selon la revendication 6, caractérisés en ce qu'il s'agit des diesters asymétriques, l'un de ces esters étant l'ester éthylique, suivants:
— éthoxycarbonyl, éthoxycarbonyl méthoxycarbonyl-11,11 endoéthano-9,10 dihydro-9,10 anthracène.
— éthoxycarbonyl, n-propoxycarbonyl-11,11 endoéthano-9,10 dihydro-9,10 anthracène
— éthoxycarbonyl, n-butoxycarbonyl-11,11 endoéthano-9,10 dihydro-9,10 anthracène
— éthoxycarbonyl, allyloxycarbonyl-11,11 endoéthano-9,10 dihydro-9,10 anthracène
— éthoxycarbonyl, n-propynyl-3 oxycarbonyl-11,11 endoéthano-9,10 dihydro-9,10 anthracène
— éthoxycarbonyl, méthoxyméthoxycarbonyl-11,11 endoéthano-9,10 dihydro-9,10 anthracène
— éthoxycarbonyl, éthoxyéthoxycarbonyl-11,11 endoéthano-9,10 dihydro-9,10 anthracène
— éthoxycarbonyl, éthoxycarbonyl, propoxycarbonyl-11,11 endoéthano-9,10 dihydro-9,10 anthracène
— éthoxycarbonyl, 2',3'-époxypropoxycarbonyl-11,11 endoéthano-9,10 dihydro-9,10 anthracène
— éthoxycarbonyl, propan-ol-3, yloxycarbonyl-11,11 endoéthano-9,10 dihydro-9,10 anthracène.

9. Nouveaux monoesters ou diesters selon la revendication 6, caractérisés en ce qu'il s'agit des diesters asymétriques, l'un de ces esters étant l'ester méthylique, suivants:
— méthoxycarbonyl, n-butoxycarbonyl-11,11 endoéthano-9,10 dihydro-9,10 anthracène
— méthoxycarbonyl, n-hexyloxycarbonyl-11,11 endoéthano-9,10 dihydro-9,10 anthracène
— méthoxycarbonyl, benzyloxycarbonyl-11,11 endoéthano-9,10 dihydro-9,10 anthracène.

10. Nouveaux monoesters ou diesters selon la revendication 6, caractérisés en ce qu'il s'agit des diesters asymétriques suivants:
— n-butoxycarbonyl, n-pentoxycarbonyl-11,11 endoéthano-9,10 dihydro-9,10 anthracène,
— n-propoxycarbonyl, n-butoxycarbonyl-11,11 endoéthano-9,10 dihydro-9,10 anthracène.

11. Nouveaux monoesters ou diesters selon la revendication 6, caractérisés en ce que $R^1$ et $R^2$ sont identiques et représentent un méthyle, n-propyle, n-butyle, isobutyle; ou sont différents et représentent éthyle et méthylène oxyméthyle ou propyle.

12. Utilisation des monoesters ou diesters de l'acide endoéthano-9,10 dihydro-9,10 anthracène dicarboxylique-11,11 tels que préparés par le procédé selon l'une quelconque des revendications 1 à 5, ou

tels que définis aux revendications 6 à 11, pour la préparation de méthylidènemalonate de formule chimique développée (I) suivante:

$$CH_2 = C\,(COOR^1)\,(COOR^2) \qquad (I)$$

dans laquelle $R^1$ et $R^2$ sont tels que précédemment définis et ne peuvent pas être H ou un atome de métal alcalin ou alcalino-terreux, par tout traitement connu en soi, notamment un traitement thermique comme une thermolyse ou pyrolyse, de préférence dans une huile minérale en présence d'anhydride maléique.

**Revendications pour les Etats contractants: ES GR**

1. Procédé de préparation de monoesters ou diesters de l'acide endoéthano-9,10 dihydro-9,10 anthracène dicarboxylique-11,11, de formule (II) suivante:

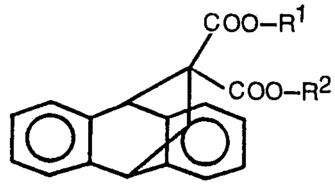

(II)

dans laquelle $R^1$ et $R^2$ peuvent être identiques ou différents et représenter H ou un atome de métal alcalin ou alcalino-terreux, notamment sodium, potassium, un groupement alkyle linéaire ou ramifié ayant de 1 à 6 atomes de carbone, alicyclique ayant de 3 à 6 atomes de carbone, alcényle ayant de 2 à 6 atomes de carbone définis dans leur variété cis ou trans, ou encore alcynyle ayant de 2 à 6 atomes de carbone, éventuellement fonctionnalisé par un ou plusieurs groupements tels que éther, époxyde, halogéno, cyano, ester, aldéhyde, cétone, aryle . . . , où $R^1$ et $R^2$ ne peuvent pas être simultanément H, caractérisé en ce qu'on fait réagir un ester malonique correspondant avec le formaldéhyde en présence d'anthracène, de manière à former ledit mono ou diester et de préférence, on sépare ce mono ou diester du milieu réactionnel, avantageusement pour être obtenu sous forme de produits cristallisés.

2. Procédé selon la revendication 1, caractérisé en ce qu'on réalise la réaction dans un milieu solvant non aqueux en présence d'un catalyseur de préférence choisi parmi l'acétate de cuivre II et l'acétate de potassium, ou encore de préférence un mélange des deux.

3. Procédé selon la revendication 2, caractérisé en ce que le solvant non aqueux est choisi parmi un solvant non miscible à l'eau ou un solvant miscible àl'eau, notamment l'acide acétique, l'anhydride acétique, le benzène, le bromobenzène, le xylène, le toluène, le diméthylformamide (DMF), le diméthylsulfoxyde (DMSO), une cétone comme la diméthylcétone ou l'éthylméthylcétone, l'acétonitrile, le dioxane, la N-méthylpyrrolidone (NMP); ou un mélange quelconque d'au moins 2 ou 3 de ces solvants.

4. Procédé selon l'une des revendications 1 à 3, caractérisé en ce que l'on prépare le monoester à partir des diesters par une hémihydrolyse en milieu basique pour donner un monosel alcalin ou alcalino-terreux d'alkoxy carbonyl-11, carboxylate-11 endoéthano-9,10 dihydro-9,10 anthracène.

5. Procédé selon la revendication 1 ou 4, caractérisé en ce qu'on prépare les diesters asymétriques en faisant réagir le monoester précité avec un dérivé halogéne approprié, par une réaction d'alkylation.

6. Procédé selon l'une des revendications 1 à 5, caractérisé en ce qu'on prépare les monoesters ou diesters dans lesquels $R^1$ et $R^2$ sont identiques et représentent un méthyle, n-propyle, n-butyle, isobutyle; ou sont différents et représentent éthyle et méthylène, oxyméthyle ou propyle.

7. Procédé selon l'une des revendications 1 à 3, caractérisé en ce qu'on prépare les diesters symétriques suivants:
— di-n-propoxycarbonyl-11,11 endoéthano-9,10 dihydro-9,10 anthracène
— di-n-butoxycarbonyl-11,11 endoéthano-9,10 dihydro-9,10 anthracène
— diméthoxycarbonyl-11,11 endoéthano-9,10 dihydro-9,10 anthracène
— diéthoxycarbonyl-11,11 endoéthano-9,10 dihydro-9,10 anthracène
— di-iso-propoxycarbonyl-11,11 endoéthano-9,10 dihydro-9,10 anthracène
— di-iso-butoxycarbonyl-11,11 endoéthano-9,10 dihydro-9,10 anthracène
— di-n-pentoxycarbonyl-11,11 endoéthano-9,10 dihydro-9,10 anthracène
— di-allyloxycarbonyl-11,11 endoéthano-9,10 dihydro-9,10 anthracène
— triméthylène-1',3'-dioxycarbonyl-11,11 endoéthano-9,10 dihydro-9,10 anthracène.

8. Procédé selon l'une des revendications 1 à 5, caractérisé en ce qu'on prépare des diesters asymétriques, l'un de ces esters étant l'ester éthylique, suivants:
— éthoxycarbonyl, éthoxycarbonyl méthoxycarbonyl-11,11 endoéthano-9,10 dihydro-9,10 anthracène.
— éthoxycarbonyl, n-propoxycarbonyl-11,11 endoéthano-9,10 dihydro-9,10 anthracène
— éthoxycarbonyl, n-butoxycarbonyl-11,11 endoéthano-9,10 dihydro-9,10 anthracène
— éthoxycarbonyl, allyloxycarbonyl-11,11 endoéthano-9,10 dihydro-9,10 anthracène
— éthoxycarbonyl, n-propynyl-3 oxycarbonyl-11,11 endoéthano-9,10 dihydro-9,10 anthracène
— éthoxycarbonyl, méthoxyméthoxycarbonyl-11,11 endoéthano-9,10 dihydro-9,10 anthracène

— éthoxycarbonyl, éthoxyéthoxycarbonyl-11,11 endoéthano-9,10 dihydro-9,10 anthracène
— éthoxycarbonyl, éthoxycarbonyl, propoxycarbonyl-11,11 endoéthano-9,10 dihydro-9,10 anthracène
— éthoxycarbonyl, 2',3'-époxypropoxycarbonyl-11,11 endoéthano-9,10 dihydro-9,10 anthracène
— éthoxycarbonyl, propan-ol-3, yloxycarbonyl-11,11 endoéthano-9,10 dihydro-9,10 anthracène.

9. Procédé selon l'une des revendications 1 à 5, caractérisé en ce qu'on prépare des diesters asymétriques, l'un de ces esters étant l'ester méthylique, suivants:
— méthoxycarbonyl, n-butoxycarbonyl-11,11 endoéthano-9,10 dihydro-9,10 anthracène
— méthoxycarbonyl, n-hexyloxycarbonyl-11,11 endoéthano-9,10 dihydro-9,10 anthracène
— méthoxycarbonyl, benzyloxycarbonyl-11,11 endoéthano-9,10 dihydro-9,10 anthracène.

10. Procédé selon l'une des revendications 1 à 5, caractérisé en ce qu'on prépare des diesters asymétriques suivants:
— n-butoxycarbonyl, n-pentoxycarbonyl-11,11 endoéthano-9,10 dihydro-9,10 anthracène,
— n-propoxycarbonyl, n-butoxycarbonyl-11,11 endoéthano-9,10 dihydro-9,10 anthracène.

11. Utilisation des monoesters ou diesters de l'acide endoéthano-9,10 dihydro-9,10 anthracène dicarboxylique-11,11 tels que préparés par le procédé selon l'une quelconque des revendications 1 à 10, pour la préparation de méthylidènemalonate de formule chimique développée (I) suivante:

$$CH_2 = C\,(COOR^1)\,(COOR^2) \qquad (I)$$

dans laquelle $R^1$ et $R^2$ sont tels que précédemment définis et ne peuvent pas être H ou un atome de métal alcalin ou alcalino-terreux, par tout traitement connu en soi, notamment un traitement thermique comme une thermolyse ou pyrolyse, de préférence dans une huile minérale en présence d'anhydride maléique.

**Patentansprüche für die Vertragsstaaten: DE GB FR IT NL SE CH LI BE AT LU**

1. Verfahren zur Herstellung von 9,10-Endoethan-9,10-dihydro-anthracen-11,11-dicarbonsäure-Monoestern oder -Diestern der folgenden Formel (II):

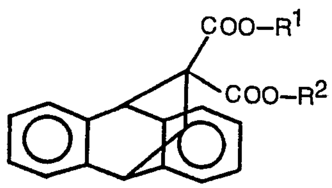
(II)

worin $R^1$ und $R^2$ gleich oder verschieden sein können und H oder ein Alkalimetall- oder Erdalkalimetallatom, insbesondere Natrium, Kalium, eine gerade oder verzweigte Alkylgruppe mit 1 bis 6 Kohlenstoffatomen, eine alicyclische Gruppe mit 3 bis 6 Kohlenstoffatomen, Alkenyl mit 2 bis 6 Kohlenstoffatomen, festgelegt in ihrer cis- oder trans-Form, oder aber auch Alkinyl mit 2 bis 6 Kohlenstoffatomen, gegebenenfalls durch eine oder mehrere Gruppen, wie Ether, Epoxid, Halogen, Cyano, Ester, Aldehyd, Keton, Aryl, ... funktionalisiert, darstellen können, wobei $R^1$ und $R^2$ nicht gleichzeitig H sein können, dadurch gekennzeichnet, daß man einen entsprechenden Malonsäureester mit dem Formaldehyd in Gegenwart von Anthracen derart reagieren läßt, daß der Mono- oder Diester gebildet wird, und man vorzugsweise diesen Mono- oder Diester aus der Reaktionsmischung abtrennt, vorteilhafterweise um sie in Form von kristallinen Produkten zu erhalten.

2. Verfahren nach Anspruch 1, dadurch gekennzeichnet, daß man die Umsetzung in einem nicht wässerigen Lösungsmittelmilieu in Gegenwart eines Katalysators, vorzugsweise ausgewählt aus Kupfer-IIacetat und Kaliumacetat oder auch vorzugsweise einer Mischung aus beiden, durchführt.

3. Verfahren nach Anspruch 2, dadurch gekennzeichnet, daß das nicht wässerige Lösungsmittel aus einem mit Wasser nicht mischbaren Lösungsmittel oder einem mit Wasser mischbaren Lösungsmittel, insbesondere Essigsäure, Essigsäureanhydrid, Benzol, Brombenzol, Xylol, Toluol, Dimethylformamid (DMF), Dimethylsulfoxid (DMSO), einem Keton, wie Dimethylketon oder Ethylmethylketon, Acetonitril, Dioxan, N-Methylpyrrolidon (NMP) oder irgendeiner Mischung aus mindestens 2 oder 3 dieser Lösungsmittel ausgewählt ist.

4. Verfahren nach einem der Ansprüche 1 bis 3, dadurch gekennzeichnet, daß man den Monoester aus Diestern durch eine Hemihydrolyse in basischem Milieu herstellt, um ein Alkali- oder Erdalkalimonosalz von Alkoxy-11-Carbonyl-11-carboxylat-9,10-endoethan-9,10-dihydro-anthracen zu liefern.

5. Verfahren nach Anspruch 1 oder 4, dadurch gekennzeichnet, daß man die asymmetrischen Diester herstellt, indem man den genannten Monoester mit einem geeigneten Halogenderivat mittels einer Alkylierungsreaktion zur Umsetzung bringt.

6. Neue 9,10-Endoethan-9,10-dihydro-anthracen-11,11-dicarbonsäure-Monoester oder -Diester, dadurch gekennzeichnet, daß sie der folgenden chemischen Strukturformel (II) entsprechen:

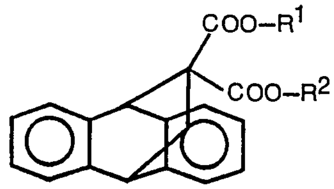

(II)

worin $R^1$ und $R^2$ gleich oder verschieden sein können und H oder ein Alkalimetall- oder Erdalkalimetallatom, insbesondere von Natrium, von Kalium, eine gerade oder verzweigte Alkylgruppe mit 1 bis 6 Kohlenstoffatomen, alicyclische Gruppen mit 3 bis 6 Kohlenstoffatomen, Alkenyle mit 2 bis 6 Kohlenstoffatomen, festgelegt in ihrer cis- oder trans-Form, oder aber auch Alkinyle mit 2 bis 6 Kohlenstoffatomen, gegebenenfalls durch eine oder mehrere Gruppen, wie Ether, Epoxid, Halogen, Cyano, Ester, Aldehyd, Keton, Aryl, ... funktionalisiert, darstellen können, wobei $R^1$ und $R^2$ nicht gleichzeitig H oder Ethyl sein können.

7. Neue Monoester oder Diester nach Anspruch 6, dadurch gekennzeichnet, daß es sich um folgende symmetrische Diester handelt:
— 11,11-Di-n-propoxycarbonyl-9,10-endoethan-9,10-dihydro-anthracen,
— 11,11-Di-n-Butoxycarbonyl-9,10-endoethan-9,10-dihydro-anthracen,
— 11,11-Dimethoxycarbonyl-9,10-endoethan-9,10-dihydro-anthracen,
— 11,11-Diethoxycarbonyl-9,10-endoethan-9,10-dihydro-anthracen,
— 11,11-Di-iso-propoxycarbonyl-9,10-endoethan-9,10-dihydro-anthracen,
— 11,11-Di-iso-butoxycarbonyl-9,10-endoethan-9,10-dihydro-anthracen,
— 11,11-Di-n-pentoxycarbonyl-9,10-endoethan-9,10-dihydro-anthracen,
— 11,11-Di-allyloxycarbonyl-9,10-endoethan-9,10-dihydro-anthracen,
— 1′,3′-Trimethylen-11,11-dioxycarbonyl-9,10-endoethan-9,10-dihydro-anthracen.

8. Neue Monoester oder Diester nach Anspruch 6, dadurch gekennzeichnet, daß es sich um folgende asymmetrische Diester handelt, wobei einer dieser Ester Ethylester ist:
— Ethoxycarbonyl-ethoxycarbonyl-11,11-methoxycarbonyl-9,10-endoethan-9,10-dihydro-anthracen,
— Ethoxycarbonyl-11,11-n-propoxycarbonyl-9,10-endoethan-9,10-dihydro-anthracen,
— Ethoxycarbonyl-11,11-n-butoxycarbonyl-9,10-endoethan-9,10-dihydro-anthracen,
— Ethoxycarbonyl-11,11-allyloxycarbonyl-9,10-endoethan-9,10-dihydro-anthracen,
— Ethoxycarbonyl-3-n-propynyl-11,11-oxycarbonyl-9,10-endoethan-9,10-dihydro-anthracen,
— Ethoxycarbonyl-11,11-methoxymethoxycarbonyl-9,10-endoethan-9,10-dihydro-anthracen,
— Ethoxycarbonyl-11,11-ethoxyethoxycarbonyl-9,10-endoethan-9,10-dihydro-anthracen,
— Ethoxycarbonyl-ethoxycarbonyl-11,11-propoxycarbonyl-9,10-endoethan-9,10-dihydro-anthracen,
— 2′,3′-Ethoxycarbonyl-11,11-epoxypropoxycarbonyl-9,10-endoethan-9,10-dihydro-anthracen,
— Ethoxycarbonyl-propan-3-ol-11,11-yloxycarbonyl-9,10-endoethan-9,10-dihydro-anthracen.

9. Neue Monoester oder Diester nach Anspruch 6, dadurch gekennzeichnet, daß es sich um folgende asymmetrische Diester handelt, wobei einer dieser Ester Methylester ist:
— Methoxycarbonyl-11,11-n-butoxycarbonyl-9,10-endoethan-9,10-dihydro-anthracen,
— Methoxycarbonyl-11,11-n-hexyloxycarbonyl-9,10-endoethan-9,10-dihydro-anthracen,
— Methoxycarbonyl-11,11-benzyloxycarbonyl-9,10-endoethan-9,10-dihydro-anthracen.

10. Neue Monoester oder Diester nach Anspruch 6, dadurch gekennzeichnet, daß es sich um folgende asymmetrische Diester handelt:
— n-Butoxycarbonyl-11,11-n-pentoxycarbonyl-9,10-endoethan-9,10-dihydro-anthracen,
— n-Propoxycarbonyl-11,11-n-butoxycarbonyl-9,10-endoethan-9,10-dihydro-anthracen.

11. Neue Monoester oder Diester nach Anspruch 6, dadurch gekennzeichnet, daß $R^1$ und $R^2$ identisch sind und Methyl, n-Propyl, n-Butyl, Isobutyl darstellen oder verschieden sind und Ethyl und Methylen, Oxymethyl oder Propyl darstellen.

12. Verwendung der nach dem Verfahren gemäß einem der Ansprüche 1 bis 5 hergestellten oder wie in den Ansprüchen 6 bis 11 definierten 9,10-Endoethan-9,10-dihydro-anthracen-11,11-dicarbonsäure-Monoester oder -Diester zur Herstellung von Methylidenmalonat der folgenden chemischen Strukturformel (I):

$$CH_2 = C\,(COOR^1)\,(COOR^2) \qquad (I).$$

worin $R^1$ und $R^2$ wie zuvor definiert sind und nicht H oder ein Alkalimetall- oder Erdalkalimetallatom sein können, durch jede an sich bekannte Behandlung, insbesondere eine Wärmebehandlung, wie Thermolyse oder Pyrolyse, vorzugsweise in einem Mineralöl in Gegenwart von Maleinsäureanhydrid.

**Patentansprüche für die Vertragsstaaten: ES GR**

1. Verfahren zur Herstellung von 9,10-Endoethan-9,10-dihydro-anthracen-11,11-dicarbonsäure-Monoestern oder -Diestern der folgenden Formel (II):

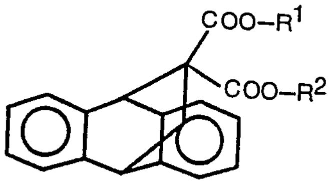

(II)

worin $R^1$ und $R^2$ gleich oder verschieden sein können und H oder ein Alkalimetall- oder Erdalkalimetallatom, insbesondere Natrium, Kalium, eine gerade oder verzweigte Alkylgruppe mit 1 bis 6 Kohlenstoffatomen, eine alicyclische Gruppe mit 3 bis 6 Kohlenstoffatomen, Alkenyl mit 2 bis 6 Kohlenstoffatomen, festgelegt in ihrer cis- oder trans-Form, oder aber auch Alkinyl mit 2 bis 6 Kohlenstoffatomen, gegebenenfalls durch eine oder mehrere Gruppen, wie Ether, Epoxid, Halogen, Cyano, Ester, Aldehyd, Keton, Aryl, ... funktionalisiert, darstellen können, wobei $R^1$ und $R^2$ nicht gleichzeitig H sein können, dadurch gekennzeichnet, daß man einen entsprechenden Malonsäureester mit dem Formaldehyd in Gegenwart von Anthracen derart reagieren läßt, daß der Mono- oder Diester gebildet wird, und man vorzugsweise diesen Mono- oder Diester aus der Reaktionsmischung abtrennt, vorteilhafterweise um sie in Form von kristallinen Produkten zu erhalten.

2. Verfahren nach Anspruch 1, dadurch gekennzeichnet, daß man die Umsetzung in einem nicht wässerigen Lösungsmittelmilieu in Gegenwart eines Katalysators, vorzugsweise ausgewählt aus Kupfer-IIacetat und Kaliumacetat oder auch vorzugsweise einer Mischung aus beiden, durchführt.

3. Verfahren nach Anspruch 2, dadurch gekennzeichnet, daß das nicht wässerige Lösungsmittel aus einem mit Wasser nicht mischbaren Lösungsmittel oder einem mit Wasser mischbaren Lösungsmittel, insbesondere Essigsäure, Essigsäureanhydrid, Benzol, Brombenzol, Xylol, Toluol, Dimethylformamid (DMF), Dimethylsulfoxid (DMSO), einem Keton, wie Dimethylketon oder Ethylmethylketon, Acetonitril, Dioxan, N-Methylpyrrolidon (NMP) oder irgendeiner Mischung aus mindestens 2 oder 3 dieser Lösungsmittel ausgewählt ist.

4. Verfahren nach einem der Ansprüche 1 bis 3, dadurch gekennzeichnet, daß man den Monoester aus Diestern durch eine Hemihydrolyse in basischem Milieu herstellt, um ein Alkali- oder Erdalkalimonosalz von Alkoxy-11-Carbonyl-11-carboxylat-9,10-endoethan-9,10-dihydro-anthracen zu liefern.

5. Verfahren nach Anspruch 1 oder 4, dadurch gekennzeichnet, daß man die asymmetrischen Diester herstellt, indem man den genannten Monoester mit einem geeigneten Halogenderivat mittels einer Alkylierungsreaktion zur Umsetzung bringt.

6. Verfahren nach einem der Ansprüche 1 bis 5, dadurch gekennzeichnet, daß man Monoester oder Diester herstellt, in denen $R^1$ und $R^2$ identisch sind und Methyl, n-Propyl, n-Butyl, Isobutyl darstellen oder verschieden sind und Ethyl und Methylen, Oxymethyl oder Propyl darstellen.

7. Verfahren nach einem der Ansprüche 1 bis 3, dadurch gekennzeichnet, daß man die folgenden symmetrischen Diester herstellt:
— 11,11-Di-n-propoxycarbonyl-9,10-endoethan-9,10-dihydro-anthracen,
— 11,11-Di-n-Butoxycarbonyl-9,10-endoethan-9,10-dihydro-anthracen,
— 11,11-Dimethoxycarbonyl-9,10-endoethan-9,10-dihydro-anthracen,
— 11,11-Diethoxycarbonyl-9,10-endoethan-9,10-dihydro-anthracen,
— 11,11-Di-iso-propoxycarbonyl-9,10-endoethan-9,10-dihydro-anthracen,
— 11,11-Di-iso-butoxycarbonyl-9,10-endoethan-9,10-dihydro-anthracen,
— 11,11-Di-n-pentoxycarbonyl-9,10-endoethan-9,10-dihydro-anthracen,
— 11,11-Di-allyloxycarbonyl-9,10-endoethan-9,10-dihydro-anthracen,
— 1',3'-Trimethylen-11,11-dioxycarbonyl-9,10-endoethan-9,10-dihydro-anthracen.

8. Verfahren nach einem der Ansprüche 1 bis 5, dadurch gekennzeichnet, daß man die folgenden asymmetrischen Diester herstellt, wobei einer dieser Ester Ethylester ist:
— Ethoxycarbonyl-ethoxycarbonyl-11,11-methoxycarbonyl-9,10-endoethan-9,10-dihydro-anthracen,
— Ethoxycarbonyl-11,11-n-propoxycarbonyl-9,10-endoethan-9,10-dihydro-anthracen,
— Ethoxycarbonyl-11,11-n-butoxycarbonyl-9,10-endoethan-9,10-dihydro-anthracen,
— Ethoxycarbonyl-11,11-allyloxycarbonyl-9,10-endoethan-9,10-dihydro-anthracen,
— Ethoxycarbonyl-3-n-propynyl-11,11-oxycarbonyl-9,10-endoethan-9,10-dihydro-anthracen,
— Ethoxycarbonyl-11,11-methoxymethoxycarbonyl-9,10-endoethan-9,10-dihydro-anthracen,
— Ethoxycarbonyl-11,11-ethoxyethoxycarbonyl-9,10-endoethan-9,10-dihydro-anthracen,
— Ethoxycarbonyl-ethoxycarbonyl-11,11-propoxycarbonyl-9,10-endoethan-9,10-dihydro-anthracen,
— 2',3'-Ethoxycarbonyl-11,11-epoxypropoxycarbonyl-9,10-endoethan-9,10-dihydro-anthracen,
— Ethoxycarbonyl-propan-3-ol-11,11-yloxycarbonyl-9,10-endoethan-9,10-dihydro-anthracen.

9. Verfahren nach einem der Ansprüche 1 bis 5, dadurch gekennzeichnet, daß man die folgenden asymmetrischen Diester herstellt, wobei einer dieser Ester Methylester ist:

— Methoxycarbonyl-11,11-n-butoxycarbonyl-9,10-endoethan-9,10-dihydro-anthracen,
— Methoxycarbonyl-11,11-n-hexyloxycarbonyl-9,10-endoethan-9,10-dihydro-anthracen,
— Methoxycarbonyl-11,11-benzyloxycarbonyl-9,10-endoethan-9,10-dihydro-anthracen.

10. Verfahren nach einem der Ansprüche 1 bis 5, dadurch gekennzeichnet, daß man die folgenden asymmetrischen Diester herstellt:
— n-Butoxycarbonyl-11,11-n-pentoxycarbonyl-9,10-endoethan-9,10-dihydro-anthracen,
— n-Propoxycarbonyl-11,11-n-butoxycarbonyl-9,10-endoethan-9,10-dihydro-anthracen.

11. Verwendung der nach dem Verfahren gemäß einem der Ansprüche 1 bis 10 hergestellten 9,10-Endoethan-9,10-dihydro-anthracen-11,11-dicarbonsäure-Monoester oder -Diester zur Herstellung von Methylidenmalonat der folgenden chemischen Strukturformel (I):

$$CH_2 = C\,(COOR^1)\,(COOR^2) \qquad (I).$$

worin $R^1$ und $R^2$ wie zuvor definiert sind und nicht H oder ein Alkalimetall- oder Erdalkalimetallatom sein können, durch jede an sich bekannte Behandlung, insbesondere eine Wärmebehandlung, wie Thermolyse oder Pyrolyse, vorzugsweise in einem Mineralöl in Gegenwart von Maleinsäureanhydrid.

**Claims for the Contracting States: DE GB FR IT NL SE CH LI BE AT LU**

1. Process for the preparation of monoesters or diesters of 9,10-endoethano-9,10-dihydroanthracene-11,11-dicarboxylic acid of the following formula (II):

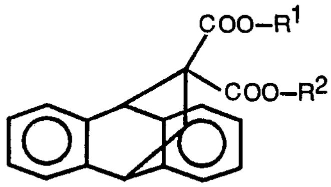
(II)

in which $R^1$ and $R^2$ can be identical or different and can represent H, an alkali metal or alkaline earth metal atom, especially sodium or potassium, a linear or branched chain alkyl group having from 1 to 6 carbon atoms, an alicyclic group having from 3 to 6 carbon atoms, an alkenyl group having from 2 to 6 carbon atoms, defined in their cis or trans variety, or an alkynyl group having from 2 to 6 carbon atoms, the said groups optionally being substituted by one or more functional groups such as ether, epoxide, halogeno, cyano, ester, aldehyde, ketone, aryl etc., where $R^1$ and $R^2$ cannot be H simultaneously, characterized in that it comprises reacting a corresponding malonic acid ester with formaldehyde, in the presence of anthracene, so as to form the said monoester or diester, and preferably separating this monoester or diester from the reaction medium so that it can be obtained, advantageously, in the form of crystalline products.

2. Process according to claim 1, characterized in that the reaction is carried out in a non-aqueous solvent medium, in the presence of a catalyst which is preferably selected from copper(II) acetate and potassium acetate or more preferably is a mixture of the two.

3. Process according to claim 2, characterized in that the non-aqueous solvent is selected from a water-immiscible solvent and a water-miscible solvent, especially acetic acid, acetic anhydride, benzene, bromobenzene, xylene, toluene, dimethylformamide (DMF), dimethyl sulfoxide (DMSO), a ketone such as dimethyl ketone or ethyl methyl ketone, acetonitrile, dioxane, N-methylpyrrolidone (NMP) or any mixture of at least 2 or 3 of these solvents.

4. Process according to one of claims 1 to 3, characterized in that the monoester is prepared from the diesters by hemihydrolysis in a basic medium to give an alkali metal or alkaline earth metal monosalt of an 11-alkoxycarbonyl-9,10-endoethano-9,10-dihydroanthracene-11-carboxylic acid.

5. Process according to claim 1 or 4, characterized in that the asymmetrical diesters are prepared by reacting the above mentioned monoester with an appropriate halogen derivative in an alkylation reaction.

6. Novel monoesters or diesters of 9,10-endoethano-9,10,-dihydroanthracene-11,11-dicarboxylic acid, characterized in that they correspond to the following structural chemical formula (II):

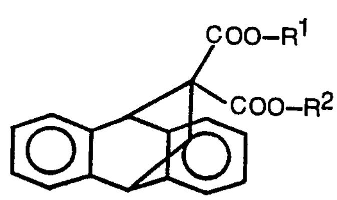
(II)

in which $R^1$ and $R^2$ can be identical or different and can represent H, an alkali metal or alkaline earth metal atom, and especially sodium or potassium, a linear or branched alkyl group having from 1 to 6 carbon

atoms, an alicyclic group having from 3 to 6 carbon atoms, an alkenyl group having from 2 to 6 carbon atoms, defined in their cis or trans variety, or an alkynyl group having from 2 to 6 carbon atoms, the said groups optionally being substituted by one or more functional groups such as ether, epoxide, halogeno, cyano, ester, aldehyde, ketone, aryl etc., where $R^1$ and $R^2$ cannot be H or ethyl simultaneously.

7. novel monoesters or diesters according to claim 6, characterized in that they are symmetrical diesters as follows:
— 11,11-di-n-propoxycarbonyl-9,10-endoethano-9,10-dihydroanthracene,
— 11,11-di-n-butoxycarbonyl-9,10-endoethano-9,10-dihydroanthracene,
— 11,11-dimethoxycarbonyl-9,10-endoethano-9,10-dihydroanthracene,
— 11,11-diethoxycarbonyl-9,10-endoethano-9,10-dihydroanthracene,
— 11,11-diisopropoxycarbonyl-9,10-endoethano-9,10-dihydroanthracene,
— 11,11-diisobutoxycarbonyl-9,10-endoethano-9,10-dihydroanthracene,
— 11,11-di-n-pentoxycarbonyl-9,10-endoethano-9,10-dihydroanthracene,
— 11,11-diallyloxycarbonyl-9,10-endoethano-9,10-dihydroanthracene, and
— 11,11-trimethylene-1',3'-dioxycarbonyl-9,10-endoethano-9,10-dihydroanthracene.

8. Novel monoeters or diesters according to claim 6, characterized in that they are asymmetrical diesters as follows, one of said esters being ethylic ester:
— 11-ethoxycarbonyl-11-ethoxycarbonylmethoxycarbonyl-9,10-endoethano-9,10-dihydroanthracene,
— 11-ethoxycarbonyl-11-n-propoxycarbonyl-9,10-endoethano-9,10-dihydroanthracene,
— 11-ethoxycarbonyl-11-n-butoxycarbonyl-9,10-endoethano-9,10-dihydroanthracene,
— 11-ethoxycarbonyl-11-allyloxycarbonyl-9,10-endoethano-9,10-dihydroanthracene,
— 11-ethoxycarbonyl-11-prop-3-ynyloxycarbonyl-9,10-endoethano-9,10-dihydroanthracene,
— 11-ethoxycarbonyl-11-methoxymethoxycarbonyl-9,10-endoethano-9,10-dihydroanthracene,
— 11-ethoxycarbonyl-11-ethoxyethoxycarbonyl-9,10-endoethano-9,10-dihydroanthracene,
— 11-ethoxycarbonyl-11-ethoxycarbonylpropoxycarbonyl-9,10-endoethano-9,10-dihydroanthracene,
— 11-ethoxycarbonyl-11-(2',3'-epoxypropoxycarbonyl)-9,10-endoethano-9,10-dihydroanthracene,
— 11-ethoxycarbonyl-11-propan-3-olyloxycarbonyl-9,10-endoethano-9,10-dihydroanthracene.

9. Novel monoesters or diesters according to claim 6, characterized in that they are asymmetrical diesters as follows, one of said esters being methyl ester:
— 11-methoxycarbonyl-11-n-butoxycarbonyl-9,10-endoethano-9,10-dihydroanthracene,
— 11-methoxycarbonyl-11-n-hexyloxycarbonyl-9,10-endoethano-9,10-dihydroanthracene,
— 11-methoxycarbonyl-11-benzyloxycarbonyl-9,10-endoethano-9,10-dihydroanthracene.

10. Novel monoesters or diesters according to claim 6, characterized in that they are asymmetrical diesters as follows:
— 11-n-butoxycarbonyl-11-n-pentoxycarbonyl-9,10-endoethano-9,10-dihydroanthracene,
— 11-n-propoxycarbonyl-11-n-butoxycarbonyl-9,10-endoethano-9,10-dihydroanthracene.

11. Novel monoesters or diesters according to claim 6, characterized in that $R^1$ and $R^2$ are identical and represent a methyl, n-propyl, n-butyl or isobutyl, or are different and represent ethyl and methyleneoxymethyl or propyl.

12. Use of the monoesters or diesters of 9,10-endoethano-9,10-dihydroanthracene-11,11-dicarboxylic acid as prepared with the process according to any one of claims 1 to 5, or such as defined in claims 6 to 11, for the preparation of methylidenemalonate of the following structural chemical formula (I):

$$CH_2=C(COOR^1)(COOR^2) \quad (I)$$

in which $R^1$ and $R^2$ are as defined above and cannot be H or an alkali metal or alkaline earth metal atom, by any treatment known per se, especially a heat treatment such as thermolysis or pyrolysis, preferably in a mineral oil, in the presence of maleic anhydride.

**Claims for the Contracting States: ES GR**

1. Process for the preparation of monoesters or diesters of 9,10-endoethano-9,10-dihydroanthracene-11,11-dicarboxylic acid of the following formula (II):

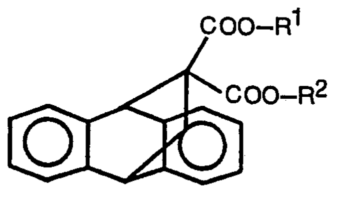

(II)

in which $R^1$ and $R^2$ can be identical or different and can represent H, an alkali metal or alkaline earth metal atom, especially sodium or potassium, a linear or branched alkyl group having from 1 to 6 carbon atoms, an alicyclic group having from 3 to 6 carbon atoms, an alkenyl group having from 2 to 6 carbon atoms, defined in their cis or trans variety, or an alkynyl group having from 2 to 6 carbon atoms, the said groups

optionally being substituted by one or more functional groups such as ether, epoxide, halogeno, cyano, ester, aldehyde, ketone, aryl etc., where $R^1$ and $R^2$ cannot be H simultaneously, characterized in that it comprises reacting a corresponding malonic acid ester with formaldehyde, in the presence of anthracene, so as to form the said monoester or diester, and preferably separating this monoester or diester from the reaction medium so that it can be obtained, advantageously, in the form of crystalline products.

2. Process according to claim 1, characterized in that the reaction is carried out in a non-aqueous solvent medium, in the presence of a catalyst which is preferably selected from copper(II) acetate and potassium acetate or more preferably is a mixture of the two.

3. Process according to claim 2, characterized in that the non-aqueous solvent is selected from a water-immiscible solvent and a water-miscible solvent, especially acetic acid, acetic anhydride, benzene, bromobenzene, xylene, toluene, dimethylformamide (DMF), dimethyl sulfoxide (DMSO), a ketone such as dimethyl ketone or ethyl methyl ketone, acetonitrile, dioxane, N-methylpyrrolidone (NMP) or any mixture of at least 2 or 3 of these solvents.

4. Process according to one of claims 1 to 3, characterized in that the monoester is prepared from the diesters by hemihydrolysis in a basic medium to give an alkali metal or alkaline earth metal monosalt of an 11-alkoxycarbonyl-9,10-endoethano-9,10-dihydroanthracene-11-carboxylic acid.

5. Process according to claim 1 or 4, characterized in that the asymmetrical diesters are prepared by reacting the above mentioned monoester with an appropriate halogen derivative in an alkylation reaction.

6. Process according to one of claims 1 to 5, characterized in that it consists in preparing the monoesters or diesters in which $R^1$ and $R^2$ are identical and represent a methyl, n-propyl, n-butyl, isobutyl; or are different and represent ethyl and methylene, oxymethyl or propyl.

7. Process according to one of claims 1 to 3, characterized in that the following symmetrical diesters are prepared:
— 11,11-di-n-propoxycarbonyl-9,10-endoethano-9,10-dihydroanthracene,
— 11,11-di-n-butoxycarbonyl-9,10-endoethano-9,10-dihydroanthracene,
— 11,11-dimethoxycarbonyl-9,10-endoethano-9,10-dihydroanthracene,
— 11,11-diethoxycarbonyl-9,10-endoethano-9,10-dihydroanthracene,
— 11,11-diisopropoxycarbonyl-9,10-endoethano-9,10-dihydroanthracene,
— 11,11-diisobutoxycarbonyl-9,10-endoethano-9,10-dihydroanthracene,
— 11,11-di-n-pentoxycarbonyl-9,10-endoethano-9,10-dihydroanthracene,
— 11,11-diallyloxycarbonyl-9,10-endoethano-9,10-dihydroanthracene, and
— 11,11-trimethylene-1',3'-dioxycarbonyl-9,10-endoethano-9,10-dihydroanthracene.

8. Process according to one of claims 1 to 5, characterized in that the following asymmetrical diesters are prepared, one of said esters being ethylic ester:
— 11-ethoxycarbonyl-11-ethoxycarbonylmethoxycarbonyl-9,10-endoethano-9,10-dihydroanthracene,
— 11-ethoxycarbonyl-11-n-propoxycarbonyl-9,10-endoethano-9,10-dihydroanthracene,
— 11-ethoxycarbonyl-11-n-butoxycarbonyl-9,10-endoethano-9,10-dihydroanthracene,
— 11-ethoxycarbonyl-11-allyloxycarbonyl-9,10-endoethano-9,10-dihydroanthracene,
— 11-ethoxycarbonyl-11-prop-3-ynyloxycarbonyl-9,10-endoethano-9,10-dihydroanthracene,
— 11-ethoxycarbonyl-11-methoxymethoxycarbonyl-9,10-endoethano-9,10-dihydroanthracene,
— 11-ethoxycarbonyl-11-ethoxyethoxycarbonyl-9,10-endoethano-9,10-dihydroanthracene,
— 11-ethoxycarbonyl-11-ethoxycarbonylpropoxycarbonyl-9,10-endoethano-9,10-dihydroanthracene,
— 11-ethoxycarbonyl-11-(2',3'-epoxypropoxycarbonyl)-9,10-endoethano-9,10-dihydroanthracene,
— 11-ethoxycarbonyl-11-propan-3-olyloxycarbonyl-9,10-endoethano-9,10-dihydroanthracene.

9. Process according to one of claims 1 to 5, characterized in that the following asymmetrical diesters are prepared, one of said esters being methyl ester:
— 11-methoxycarbonyl-11-n-butoxycarbonyl-9,10-endoethano-9,10-dihydroanthracene,
— 11-methoxycarbonyl-11-n-hexyloxycarbonyl-9,10-endoethano-9,10-dihydroanthracene,
— 11-methoxycarbonyl-11-benzyloxycarbonyl-9,10-endoethano-9,10-dihydroanthracene.

10. Process according to one of claims 1 to 5, characterized in that the following asymmetrical diesters are prepared:
— 11-n-butoxycarbonyl-11-n-pentoxycarbonyl-9,10-endoethano-9,10-dihydroanthracene,
— 11-n-propoxycarbonyl-11-n-butoxycarbonyl-9,10-endoethano-9,10-dihydroanthracene.

11. Use of the monoesters or diesters of 9,10-endoethano-9,10-dihydroanthracene-11,11-dicarboxylic acid as prepared with the process according to any one of claims 1 to 5, or such as defined in claims 6 to 11, for the preparation of methylidenemalonate of the following structural chemical formula (I):

$$CH_2=C(COOR^1)(COOR^2) \quad (I)$$

in which $R^1$ and $R^2$ are as defined above and cannot be H or an alkali metal or alkaline earth metal atom, by any treatment known per se, especially a heat treatment such as thermolysis or pyrolysis, preferably in a mineral oil, in the presence of maleic anhydride.